# EUROPÄISCHE PATENTSCHRIFT

(11) **EP 2 836 474 B1**
(45) Veröffentlichungstag und Bekanntmachung des Hinweises auf die Patenterteilung: **31.01.2018**
(21) Anmeldenummer: 13714961.3
(22) Anmeldetag: 11.04.2013
(51) Int. Cl.: C07C 45/50, C07C 47/02

(54) **VERFAHREN ZUR ERGÄNZUNG DES KATALYSATORS BEI DER KONTINUIERLICHEN HYDROFORMYLIERUNG**
METHOD FOR REPLENISHING THE CATALYST IN CONTINUOUS HYDROFORMYLATION
PROCÉDÉ POUR COMPLÉTER LE CATALYSEUR LORS DE L'HYDROFORMYLATION CONTINUE

(30) Priorität: 12.04.2012 EP 12163918
(43) Veröffentlichungstag der Anmeldung: 18.02.2015
(73) Patentinhaber: BASF SE, 67056 Ludwigshafen am Rhein (DE)
(72) Erfinder: ZELLER, Edgar, 68165 Mannheim (DE); ULONSKA, Armin, 67150 Niederkirchen (DE); HÖHN, Arthur, 67281 Kirchheim (DE); UHRIG, Volker, 67133 Maxdorf (DE); MAGNIE, Rene, 67127 Rödersheim-Gronau (DE); FATTLER, Reimund, 67725 Börrstadt (DE)
(86) Internationale Anmeldenummer: PCT/EP2013/057526
(87) Internationale Veröffentlichungsnummer: WO 2013/153136

(56) Entgegenhaltungen:
- US-A- 4 247 486
- US-B2- 6 700 021

## Beschreibung

### HINTERGRUND DER ERFINDUNG

Die vorliegende Erfindung betrifft ein Verfahren zur Ergänzung des Katalysators bei der kontinuierlichen Hydroformylierung im laufenden Betrieb.

### STAND DER TECHNIK

Die kontinuierliche Hydroformylierung von Olefinen unter Verwendung eines Rhodium-Katalysatorsystems ist ein wohlbekanntes ausgereiftes Verfahren zur kommerziellen Herstellung von Oxo-Aldehyden und deren Hydrierungsprodukten, den Oxo-Alkoholen. Das Verfahren ist in zahlreichen Patentdokumenten beschrieben, z. B. in der US 3,527,809, US 4,148,830, US 4,247,486 und US 4,247,468.

Bei kontinuierlicher Arbeitsweise nimmt im Laufe der Zeit die Aktivität des Katalysatorsystems ab. Um die ursprüngliche Aktivität des Katalysatorsystems und die Produktivität des Reaktorsystems aufrecht zu erhalten, muss man die unwirksam gewordenen Katalysatorbestandteile in Abständen durch Zugabe frischer Übergangsmetallverbindungen und/oder frischer Liganden ersetzen, aus denen unter den Hydroformylierungsbedingungen die aktive Katalysatorspezies nachgebildet wird. Da die Hydroformylierung eine stark exotherme Reaktion ist, muss dabei ein Durchgehen der Reaktion in Folge der erhöhten Katalysatoraktivität auf jeden Fall vermieden werden.

Derzeit geht man bei der Zugabe frischer Übergangsmetallverbindungen, wie z. B. bei der Zugabe von Rhodiumacetat, so vor, dass zuvor die Reaktorbelastung, die zugeführte Menge an Ausgangsolefin, die Reaktortemperatur und/oder der Flüssigkeitsstand im Reaktor verringert wird, bevor innerhalb einer kurzen Zeitspanne von typischerweise 2 bis 5 Stunden die Übergangsmetallverbindung zugegeben wird. Durch diese Vorgehensweise lässt sich die mit der Zugabe verbundene Wärmeentwicklung und die damit verbundene Temperaturerhöhung im Reaktor gut kontrollieren und ein Durchgehen der Reaktion wirkungsvoll verhindern (z. B. in dem US 6,700,021). Nachteilig an dieser Vorgehensweise ist jedoch, dass die Kapazität der Hydroformylierungsanlage während der Ergänzung der Übergangsmetallverbindung nicht voll ausgenutzt wird, was zu entsprechenden Produktionsverlusten führt.

Der vorliegenden Erfindung liegt die Aufgabe zu Grunde, ein verbessertes Verfahren zur Herstellung von gesättigten C₃-C₂₀-Alkoholen aus C₃-C₂₀-Aldehyden zur Verfügung zu stellen, bei dem eine sichere Ergänzung der Übergangsmetallverbindung mit möglichst geringen Produktionsverlusten verbunden ist.

Überraschenderweise wurde nun gefunden, dass sich bei der kontinuierlichen Hydroformylierung die Übergangsmetallquelle ergänzen lässt, ohne dass ein stark verringerter Umsatzgrad bzw. eine stark verringerte Ausbeute in Kauf genommen werden muss, wenn die Zugabegeschwindigkeit der Übergangsmetallquelle in die Reaktionszone in Abhängigkeit von der Raum-Zeit-Ausbeute gesteuert wird. Somit gelingt es, bei im Wesentlichen voller Auslastung des Reaktors, die Aktivität des Katalysators in dem gewünschten Zielbereich zu halten, wobei die Zugabe der Übergangsmetallquelle nur eine marginale, technisch leicht beherrschbare Temperaturerhöhung im Reaktor hervorruft.

### ZUSAMMENFASSUNG DER ERFINDUNG

Gegenstand der Erfindung ist ein Verfahren zur kontinuierlichen Hydroformylierung, bei dem ein Olefinausgangsmaterial, welches wenigstens ein Olefin mit 3 bis 20 Kohlenstoffatomen enthält, bei erhöhter Temperatur und bei erhöhtem Druck in einer Reaktionszone mit Synthesegas in Gegenwart eines mit einem Organophosphorliganden komplexierten, homogenen Übergangsmetallkatalysators und freiem Liganden umgesetzt wird, wobei der Katalysator in situ in der Reaktionszone gebildet wird und man der Reaktionszone zum Ausgleich von Katalysatorverlusten eine Lösung einer Übergangsmetallquelle zufügt, das dadurch gekennzeichnet, dass man die Raum-Zeit-Ausbeute der Reaktionszone an Hydroformylierungsprodukt bestimmt und die Zugabegeschwindigkeit der Übergangsmetallquelle in die Reaktionszone in Abhängigkeit von der Raum-Zeit-Ausbeute steuert, wobei man
- ein Stellmittel zum Einstellen der Zugabegeschwindigkeit der Übergangsmetallquelle in die Reaktionszone vorsieht,
- einen Zielwert für die Raum-Zeit-Ausbeute der Reaktionszone an Hydroformylierungsprodukt festlegt,
- den Istwerts für die Raum-Zeit-Ausbeute ermittelt,
- nach Erreichen eines unteren Grenzwerts für die Abweichung des Istwerts vom Zielwert die Menge an Übergangsmetallquelle ermittelt, die erforderlich ist, den Katalysatorverlust auszugleichen, und
- der Reaktionszone eine Lösung der Übergangsmetallquelle zufügt, wobei die Zugabegeschwindigkeit der Übergangsmetallquelle in die Reaktionszone so gesteuert wird, dass die Raum-Zeit-Ausbeute einen oberen Grenzwert für die Abweichung des Istwerts vom Zielwert nicht überschreitet.

### BESCHREIBUNG DER ERFINDUNG

"Katalysatorverluste" bezeichnet im Sinne der Erfindung ganz allgemein die Abnahme des aktiven Katalysators in der Reaktionszone. Diese kann beispielsweise durch Deaktivierung, d. h. die Bildung geringer oder nicht mehr aktiver Übergangsmetallkomplexe oder -verbindungen, erfolgen. Diese kann beispielsweise auch dadurch erfolgen, dass im Reaktionsaustrag enthaltener Katalysator nicht vollständig in die Reaktionszone zurückgeführt werden kann.

"Übergangsmetallquelle" bezeichnet ganz allgemein Übergangsmetalle, Übergangsmetallverbindungen und Übergangsmetallkomplexe, woraus in der Reaktionszone unter den Hydroformylierungsbedingungen der Hydroformylierungskatalysator in situ gebildet wird. Bei der Übergangsmetallquelle handelt es sich vorzugsweise nicht um außerhalb der Reaktionszone bereitgestellten präformierten Katalysator.

Das Übergangsmetall ist vorzugsweise ein Element der 9. Gruppe des Periodensystems der Elemente und besonders bevorzugt Rhodium oder Cobalt. Insbesondere wird als Übergangsmetall Rhodium eingesetzt.

Als Übergangsmetallquelle geeignete Rhodiumverbindungen oder-komplexe sind z. B. Rhodium(II)- und Rhodium(III)-salze, wie Rhodium(II)- bzw. Rhodium(III)-carboxylat, Rhodium(II)- und Rhodium(III)-acetat, etc. Weiterhin eignen sich Rhodiumkomplexe, wie Rhodiumbiscarbonylacetylacetonat, Acetylacetonatobisethylenrhodium(I) Acetyl-acetonatocyclooctadienylrhodium(I), Acetylacetonatonorbornadienylrhodium(I), Acetyl-acetonatocarbonyltriphenylphosphinrhodium(I), etc. Bevorzugt wird als Übergangsmetallquelle Rhodium(II)- und/oder Rhodium(III)-acetat eingesetzt.

Als Übergangsmetallquelle geeignete Cobaltverbindungen sind z. B. Cobalt(II)sulfat, Cobalt(II)carbonat, deren Amin- oder Hydratkomplexe, Cobaltcarboxylate, wie Cobaltacetat, Cobaltethylhexanoat, Cobaltnaphthanoat, und Cobaltcaproat. Geeignet sind auch Carbonylkomplexe des Cobalts, wie Dicobaltoctacarbonyl, Tetracobaltdodecacarbonyl und Hexacobalthexadecacarbonyl.

Die genannten und weitere geeignete Übergangsmetallverbindungen und -komplexe sind im Prinzip bekannt und in der Literatur hinreichend beschrieben, oder sie können vom Fachmann analog zu den bereits bekannten Verbindungen hergestellt werden.

Besonders bevorzugt wird als Übergangsmetallquelle Rhodium(II)- und/oder Rhodium(III)-acetat eingesetzt.

In einer bevorzugten Ausführung des erfindungsgemäßen Verfahrens wird der Reaktionszone eine Lösung der Übergangsmetallquelle in einem Lösungsmittel zugefügt, das ausgewählt ist unter Wasser, C₁-C₄-Alkanolen und Mischungen davon.

Insbesondere wird der Reaktionszone eine Lösung der Übergangsmetallquelle in Wasser zugefügt. Speziell wird der Reaktionszone eine Lösung von Rhodium(II)- und/oder Rhodium(III)-acetat in Wasser als alleinigem Lösungsmittel zugefügt.

Bevorzugt beträgt die Konzentration der Übergangsmetallquelle in der Lösung, die der Reaktionszone zugeführt wird, 0,1 bis 10 Gew.-%, besonders bevorzugt 0,2 bis 7,5 Gew.-%, insbesondere 0,5 bis 5 Gew.-%.

Bevorzugt wird zur Hydroformylierung ein homogen im Reaktionsmedium der Hydroformylierungsreaktion löslicher Übergangsmetall-Komplex-Katalysator eingesetzt, der eine oder mehrere Organophosphorverbindung(en) als Liganden aufweist. Bevorzugte Liganden sind Phosphinliganden aus der Klasse der Triarylphosphine, C₁-C₆-Alkyldiarylphosphine oder Arylalkyldiphosphine und insbesondere Triphenylphosphin.

Des Weiteren geeignete Liganden sind Diphosphitverbindungen, wie sie z. B. in der EP 0 214 622 A2, US 4,668,651, US 4,748,261, US 4,769,498, US 4,885,401, US 5,235,113, US 5,391,801, US 5,663,403, US 5,728,861 und US 6,172,267 beschrieben sind.

Geeignete sterisch gehinderte Liganden sind z. B. in US-A 4 774 361, US-A 4 835 299, US-A 5 059 710, US-A 5 113 022, US-A 5 179 055, US-A 5 260 491, US-A 5 264 616, US-A 5 288 918, US-A 5 360 938, EP-A 472 071 und EP-A 518 241 beschrieben.

In einer besonders bevorzugten Ausführungsform des erfindungsgemäßen Verfahrens wird ein Rhodiumkatalysator eingesetzt, der Triphenylphosphin als Liganden umfasst.

Bevorzugt liegt die Übergangsmetallkonzentration in der Reaktionszone in einem Bereich von 150 bis 250 ppm, besonders bevorzugt von 180 bis 200 ppm, bezogen auf den gesamten flüssigen Inhalt der Reaktionszone.

Bevorzugt liegt das Molmengenverhältnis von Ligand zu Übergangsmetall in der Reaktionszone in einem Bereich von 100 : 1 bis 1000 : 1, vorzugsweise 200 : 1 bis 500 : 1, insbesondere 300 : 1 bis 500 : 1.

Das eingesetzte Wasserstoff und Kohlenmonoxid enthaltende Gasgemisch wird üblicherweise als Synthesegas bezeichnet. Die Zusammensetzung des Synthesegases kann in weiten Bereichen variieren. Das molare Verhältnis von Kohlenmonoxid und Wasserstoff beträgt in der Regel 2 : 1 bis 1 : 2, insbesondere 45 : 55 bis 50 : 50.

Die Hydroformylierung kann in einem geeigneten, unter den jeweiligen Reaktionsbedingungen inerten Lösungsmittel durchgeführt werden. Geeignete Lösungsmittel sind z. B. die bei der Hydroformylierung gebildeten Aldehyde und höher siedende Reaktionskomponenten, z. B. die Produkte der Aldolkondensation. Weiterhin geeignet sind Aromaten, wie Toluol und Xylole, Kohlenwasserstoffe oder Gemische von Kohlenwasserstoffen, Ester aliphatischer Carbonsäuren mit Alkanolen, beispielsweise Texanol®, und Ester aromatischer Carbonsäuren , z. B. C₈-C₁₃-Dialkylphthalate.

Geeignete Olefin-Einsatzmaterialien für das erfindungsgemäße Hydroformylierungsverfahren sind prinzipiell alle Verbindungen, welche eine oder mehrere ethylenisch ungesättigte Doppelbindungen enthalten. Dazu zählen Olefine mit endständigen und mit innenständigen Doppelbindungen, geradkettige und verzweigte Olefine, cyclische Olefine sowie Olefine, die unter den Hydroformylierungsbedingungen im Wesentlichen inerte Substituenten aufweisen. Bevorzugt sind Olefin-Einsatzmaterialien, die Olefine mit 2 bis 12, besonders bevorzugt mit 3 bis 8 Kohlenstoffatomen enthalten.

Geeignete α-Olefine sind z. B. Ethylen, Propen, 1-Buten, 1-Penten, 1-Hexen, 1-Hepten, 1-Octen, 1-Nonen, 1-Decen, 1-Undecen, 1-Dodecen, etc. Bevorzugte verzweigte interne Olefine sind C₄-C₂₀-olefine, wie 2-Methyl-2-buten, 2-Methyl-2-penten, 3-Methyl-2-penten, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische, etc. Geeignete Olefine sind weiterhin C₅-C₈-Cycloalkene, wie Cyclopenten, Cyclohexen, Cyclohepten, Cycloocten und deren Derivate, wie z. B. deren C₁-C₂₀-Alkylderivate mit 1 bis 5 Alkylsubstituenten. Geeignete Olefine sind weiterhin Vinylaromaten, wie Styrol, α-Methylstyrol, 4-Isobutylstyrol etc. Geeignete Olefine sind weiterhin die Ester, Halbester und Amide α,β-ethylenisch ungesättigte Mono- und/oder Dicarbonsäuren, wie 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, Vinylether, wie Vinylmethylether, Vinylethylether, Vinylpropylether, etc., Vinylchlorid, Allylchlorid, C₃-C₂₀-Alkenole,-Alkendiole und -Alkadienole, wie Allylalkohol, Hex-1-en-4-ol, Oct-1-en-4-ol, 2,7-Octadienol-1. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, 1,8-Nonadien, 1,9-Decadien, 1,10-Undecadien, 1,11-Dodecadien, 1,12-Tridecadien, 1,13-Tetradecadien, Vinylcyclohexen, Dicyclopentadien, 1,5,9-Cyclooctatrien sowie Butadienhomo- und - copolymere.

Das erfindungsgemäße Verfahren eignet sich speziell für Verfahren zur kontinuierlichen Hydroformylierung, bei denen ein Olefinausgangsmaterial eingesetzt wird, welches wenigstens ein Olefin mit 3 oder 4 Kohlenstoffatomen enthält. Speziell bei diesen Verfahren ist Hydroformylierung mit einer deutlichen Wärmetönung bei der Zugabe der Rhodiumquelle verbunden.

In einer speziellen Ausführung wird in dem Hydroformylierungsverfahren ein technisch zur Verfügung stehendes olefinhaltiges Kohlenwasserstoffgemisch eingesetzt.

Bevorzugt wird in dem erfindungsgemäßen Verfahren ein propenhaltiges Kohlenwasserstoffgemisch eingesetzt. Ein bevorzugtes technisches propenhaltiges Kohlenwasserstoffgemisch ist der C₃-Schnitt. Als Ausgangsmaterial geeignete Propenströme können neben Propen auch Propan enthalten. Der Propangehalt beträgt z. B. 0,5 bis 40 Gew.-%, speziell 1 bis 20 Gew.-%, Propan. In einer speziellen Ausführungsform beträgt der Propangehalt maximal 10 Gew.-%, besonders bevorzugt maximal 6 Gew.-%, bezogen auf das gesamte propenhaltige Kohlenwasserstoffgemisch.

Ein weiteres bevorzugtes technisches Olefingemisch ist der C₄-Schnitt. C₄-Schnitte sind beispielsweise durch Fluid Catalytic Cracking oder Steamcracken von Gasöl bzw. durch Steamcracken von Naphtha erhältlich. Je nach Zusammensetzung des C₄-Schnitts unterscheidet man den Gesamt-C₄-Schnitt (Roh-C₄-Schnitt), das nach der Abtrennung von 1,3-Butadien erhaltene so genannte Raffinat I sowie das nach der Isobutenabtrennung erhaltene Raffinat II. Raffinat II ist als olefinhaltiges Kohlenwasserstoffgemisch für die Hydroformylierung besonders geeignet.

Die Hydroformylierung erfolgt in einer Reaktionszone, die einen oder mehrere, gleiche oder verschiedene Reaktoren umfassen kann. Im einfachsten Fall wird die Reaktionszone von einem einzelnen Reaktor gebildet. Die Reaktoren können jeweils gleiche oder verschiedene Vermischungscharakteristiken aufweisen. Die Reaktoren können gewünschtenfalls durch Einbauten ein- oder mehrfach unterteilt sein. Bilden zwei oder mehrere Reaktoren eine Zone, so können diese untereinander beliebig verschaltet sein, z. B. parallel oder in Reihe. Als Reaktoren können prinzipiell sämtliche für Hydroformylierungsreaktionen geeignete Reaktortypen verwendet werden, beispielsweise Rührreaktoren, Blasensäulenreaktoren, wie sie z. B. in US-A 4,778,929 beschrieben sind, Umlaufreaktoren, wie sie z. B. Gegenstand von EP-A 1 114 017 sind, Rohrreaktoren, wobei die einzelnen Reaktoren einer Reihe unterschiedliche Mischungscharakteristiken haben können, wie z. B. in EP-A 423 769 beschrieben, des Weiteren können kompartimentierte Reaktoren verwendet werden, wie sie z. B. Gegenstand von EP-A 1 231 198 oder von US-A 5,728,893 sind.

Die Temperatur in der Reaktionszone liegt im Allgemeinen in einem Bereich von etwa 50 bis 200 °C, vorzugsweise etwa 60 bis 180 °C, insbesondere etwa 70 bis 160 °C. Die Umsetzung wird vorzugsweise bei einem Druck im Bereich von etwa 10 bis 700 bar, vorzugsweise 15 bis 200 bar, insbesondere 15 bis 60 bar durchgeführt.

Die Bestimmung der Raum-Zeit-Ausbeute erfolgt vorzugsweise über die Messung des Aldehydgehalts im Austrag aus der Reaktionszone. Diese Messung erfolgt vorzugsweise durch eine Online-Messvorrichtung. Möglich ist jedoch auch, in regelmäßigen Abständen aus dem Austrag aus der Reaktionszone Proben zu entnehmen und den Aldehydgehalt in einer separaten Analysevorrichtung zu bestimmen. Die Bestimmung des Aldehydgehalts kann z. B. durch Gaschromatographie, Infrarotspektroskopie, Kolorimetrie oder Chemilumineszenzanalyse erfolgen. So ist es z. B. zur quantitativen Chemilumineszenzanalyse möglich, den Aldehydgehalt nach der Umwandlung des Aldehyds in ein fluoreszierendes Derivat, z. B. unter Anwendung von Fluoral-P (4-Amino-3-penten-2-on), mit hoher Genauigkeit zu bestimmen.

Im einfachsten Fall des erfindungsgemäßen Verfahrens wird abhängig von der Raum-Zeit-Ausbeute (RZA) die Zugabegeschwindigkeit der Übergangsmetallquelle in die Reaktionszone mittels eines geeigneten Stellmittels (Aktors) eingestellt.

Im einfachsten Fall des erfindungsgemäßen Verfahrens kann dabei auf eine Rückkopplung der Ausgangsgröße (RZA) auf die Zugabegeschwindigkeit im Sinne einer Regelung verzichtet werden. So kann z. B. nach Erreichen eines unteren Grenzwerts für die Raum-Zeit-Ausbeute der Reaktionszone eine Lösung der Übergangsmetallquelle so zugefügt werden, dass die damit erzielte Erhöhung der Übergangsmetallkonzentration im Reaktor einen zuvor festgelegten Grenzwert nicht überschreitet. Somit lässt sich ein Durchgehen der Reaktion in der Regel sicher vermeiden.

Bevorzugt liegt die Übergangsmetallkonzentration in der Reaktionszone in einem Bereich von 150 bis 250 ppm, besonders bevorzugt von 180 bis 200 ppm, bezogen auf den gesamten flüssigen Inhalt der Reaktionszone. Speziell handelt es sich bei dem Übergangsmetall um Rhodium. Dabei bezieht sich die Übergangsmetallkonzentration nur auf den Anteil an "aktivem" Übergangsmetall, d. h. in katalytisch aktiver Form oder eine darin unter den Reaktionsbedingungen überführbare Form.

Bevorzugt liegt die Zugabegeschwindigkeit der Übergangsmetallquelle in die Reaktionszone in einem Bereich von 0,1 ppm bis 10 ppm Übergangsmetallquelle pro Tag, besonders bevorzugt in einem Bereich von 0,5 ppm bis 5 ppm Übergangsmetallquelle pro Tag, bezogen auf den gesamten flüssigen Inhalt der Reaktionszone. Dies gilt sowohl für die gesteuerte als auch die geregelte Variante des erfindungsgemäßen Verfahrens. Speziell handelt es sich bei dem Übergangsmetall um Rhodium. Speziell handelt es sich bei der Übergangsmetallquelle um Rhodiumacetat.

Bevorzugt beträgt der durch die Zuführung der Rhodiumquelle bedingte Temperaturanstieg in der Reaktionszone weniger als 2 K, bevorzugt weniger als 1 K, insbesondere weniger als 0,5 K. Dies gilt sowohl für die gesteuerte als auch die geregelte Variante des erfindungsgemäßen Verfahrens.

Geeignete Vorrichtungen zur Steuerung der Zugabegeschwindigkeit der Übergangsmetallquelle in die Reaktionszone sind ausgewählt unter Durchflussmengenbegrenzern, Dosierventilen und Dosierpumpen. In einer geeigneten Ausführungsform wird zur Steuerung der Zugabegeschwindigkeit eine Kombination aus wenigstens einer Pumpe und wenigstens einem Durchflussmengenbegrenzer und/oder wenigstens einem Dosierventil eingesetzt. In einer bevorzugten Ausführungsform wird zur Steuerung der Zugabegeschwindigkeit der Übergangsmetallquelle in die Reaktionszone wenigstens eine Dosierpumpe eingesetzt. Die Dosierpumpe übernimmt die Funktionen aller sonst notwendigen Systemkomponenten, wie Pumpe, Dosierventil, gegebenenfalls Absperrventil, etc., auf einmal. Geeignete Dosierpumpen sind dem Fachmann bekannt und haben die Aufgabe, genau bemessene Flüssigkeitsmengen zu fördern. Dabei kann es sich z. B. um Membranpumpen oder um Kolbenpumpen handeln.

In einer bevorzugten Ausführungsform umfasst das erfindungsgemäße Verfahren eine Regelung der Zugabegeschwindigkeit der Übergangsmetallquelle in die Reaktionszone nach Maßgabe des jeweiligen Istwerts für die RZA, wie zuvor beschrieben. Die zuvor genannten Vorrichtungen zur Steuerung der Zugabegeschwindigkeit sind auch geeignete Stellmittel (Aktoren) im Falle einer geregelten Ausführung des erfindungsgemäßen Verfahrens. Dann setzen sie beispielsweise die elektronischen Signale (z. B. vom Steuerungscomputer ausgehende Befehle) in eine entsprechende mechanische Erhöhung oder Verringerung der Zuflussgeschwindigkeit der Lösung der Übergangsmetallquelle in die Reaktionszone um und greifen so regulierend in das erfindungsgemäß eingesetzte Regelungssystem ein.

Bevorzugt beträgt der untere Grenzwert für die Abweichung des Istwerts für die Raum-Zeit-Ausbeute vom Zielwert maximal 40 %, besonders bevorzugt maximal 30 %, bezogen auf den Zielwert.

Bevorzugt beträgt der obere Grenzwert für die Abweichung des Istwerts für die Raum-Zeit-Ausbeute vom Zielwert maximal 10 %, besonders bevorzugt maximal 5 %, bezogen auf den Zielwert.

Die Abtrennung des Hydroformylierungsproduktes vom Austrag aus der Reaktionszone kann auf verschiedene Weise erfolgen. Es kann zum Beispiel ein Hydroformylierungsverfahren mit Flüssigaustrag zum Einsatz kommen, wie es z. B. in der US-A 4,148,830, EP-A 16 286, EP-A 188 246 oder EP-A 423 769 beschrieben ist. Bevorzugt ist ein Flüssigaustragsverfahren, bei dem der im Wesentlichen, bis auf das im Überschuss zur Hydroformylierung eingesetzte Synthesegas, flüssige Austrag aus der Reaktionszone entspannt wird, wobei infolge der Druckerniedrigung der Austrag in eine Flüssigphase, die im Wesentlichen aus hochsiedenden Nebenprodukten, dem homogen gelösten Hydroformylierungskatalysator, einem Teil des Hydroformylierungsprodukts und gelöstem, nicht umgesetztem Propylen und Propan besteht, und eine Gasphase, die im Wesentlichen aus Hydroformylierungsprodukt, nicht umgesetztem Propylen und Propan sowie nicht umgesetztem Kohlenmonoxid und Wasserstoff sowie Inerten (z. B. N₂, CO₂, Methan) besteht, aufgetrennt wird. Die Flüssigphase kann, gegebenenfalls nach weiterer Abtrennung darin enthaltener Produktaldehyde, als Rückführstrom wieder in den Reaktor geleitet werden. Durch zumindest partielle Kondensation der Gasphase wird das rohe Hydroformylierungsprodukt erhalten. Die bei der Kondensation zurückbleibende Gasphase wird ganz oder teilweise in die Reaktionszone zurückgeführt. Mit Vorteil kann die in der Entspannungsstufe primär anfallende Gas- und Flüssigphase nach dem in der WO 97/07086 beschriebenen Verfahren aufgearbeitet werden. Zu diesem Zweck wird die Flüssigphase erwärmt und in den oberen Bereich einer Kolonne eingeleitet, während die Gasphase in den Sumpf der Kolonne eingeleitet wird. Flüssigphase und Gasphase werden dadurch im Gegenstrom geführt. Durch den innigen Kontakt der Gasphase mit der Flüssigphase werden die in der Flüssigphase vorhandenen Restmengen an Hydroformylierungsprodukt, nicht umgesetztem Propylen und Propan in die Gasphase transferiert, so dass der die Kolonne am Kopf verlassende Gasstrom im Vergleich zu dem am unteren Ende der Kolonne eingeführten Gasstrom an Hydroformylierungsprodukt sowie nicht umgesetztem Propen und Propan angereichert ist.

Bevorzugt beträgt bei Entnahme eines flüssigen Austrags aus der Reaktionszone der Zielwert für die Raum-Zeit-Ausbeute der Reaktionszone an Hydroformylierungsprodukt 75 bis 120 kg/m³h, besonders bevorzugt 85 bis 110 kg/m³h, insbesondere 94 bis 100 kg/m³h.

Alternativ kann man nach dem so genannten Kreisgasverfahren vorgehen, bei dem aus dem Gasraum des Hydroformylierungsreaktors ein Gasstrom abgezogen wird. Dieser Gasstrom besteht im Wesentlichen aus Synthesegas, nicht umgesetztem Propylen und Propan, wobei nach Maßgabe des Dampfdrucks im Hydroformylierungsreaktor das bei der Hydroformylierungsreaktion gebildete Hydroformylierungsprodukt mitgeführt wird. Aus dem Gasstrom wird das mitgeführte rohe Hydroformylierungsprodukt z. B. durch Abkühlen auskondensiert, und der vom Flüssiganteil befreite Gasstrom wird zurück in den Hydroformylierungsreaktor geführt. Das im auskondensierten rohen Hydroformylierungsprodukt gelöst enthaltene, nicht umgesetzte Propylen und Propan kann dann, wie beschrieben, z. B. in einer Entgasungskolonne freigesetzt werden.

Bevorzugt beträgt bei Entnahme eines gasförmigen Austrags aus der Reaktionszone der Zielwert für die Raum-Zeit-Ausbeute der Reaktionszone an Hydroformylierungsprodukt 50 bis 75 kg/m³h, besonders bevorzugt 55 bis 70 kg/m³h, insbesondere 60 bis 65 kg/m³h.

Die Erfindung wird anhand der folgenden, nicht einschränkenden Beispiele näher erläutert.

### Beispiele:

### Beispiel 1 (Vergleichsbeispiel mit konventioneller Rhodium-Ergänzung):

Zum Reaktor eines laufenden Prozesses der Hydroformulierung von Propylen mit Rhodium-Triphenylphosphin-Carbonyl-Komplex als Katalysator und gasförmigem Reaktionsaustrag wurde nach Abfall der Raum-Zeit-Ausbeute auf einen Wert von etwa 45 kg/m³h frisches Rhodiumacetat zugegeben. Dazu wurde vorab die Anlagenauslastung (Propylen-Durchsatz) von 100 % auf ca. 60 % reduziert, der Propylengehalt im Kreisgas von ca. 40 Vol.% auf ca. 30 Vol.% verringert, der Flüssigkeitsstand im Reaktor um ca. 30 % und die Reaktortemperatur von 109 °C auf 105 °C gesenkt, bevor 50 l einer 5 Gew.-% wässrigen Rhodiumacetatlösung in einer Zeitspanne von ca. 2 h durch eine Pumpe zudosiert wurden. Dabei wurde eine Temperaturerhöhung von bis zu 4 °C beobachtet, die durch vorhandene Wärmeabfuhrmöglichkeiten gut geregelt werden konnte. Danach wurde die Anlagenauslastung wieder auf 100 % gebracht und die Betriebsbedingungen entsprechend angepasst. Die niedrigere erforderliche Temperatur (107 °C anstatt 109 °C) für die volle Auslastung reflektierte eine höhere Aktivität des Katalysators nach der Ergänzung. Der ganze Rhodiumergänzungsprozess dauerte ca. 18 h.

### Beispiel 2 (erfindungsgemäße kontinuierliche Rhodium-Dosierung):

Zu einem voll ausgelasteten Reaktor eines kontinuierlich betriebenen Prozesses der Hydroformulierung von Propylen mit Rhodium-Triphenylphosphin-Carbonyl-Komplex als Katalysator und gasförmigem Reaktionsaustrag wurden nach Abfall der Raum-Zeit-Ausbeute auf einen Wert von etwa 45 kg/m³h 100 I einer 2,5 Gew.-% wässrigen Rhodiumacetatlösung durch eine Dosierpumpe in einer Zeitspanne von 10 Tagen zudosiert. Über den Dosierzeitraum wurde kein nennenswerter Temperaturanstieg im Reaktor beobachtet. Die erforderliche Reaktortemperatur für eine volle Auslastung konnte nach der Rhodium-Ergänzung um ca. 1 °C gesenkt werden, was auf eine Erhöhung der Katalysatoraktivität hindeutet. Die Raum-Zeit-Ausbeute lag nach Abschluss der Zugabe des Rhodiumacetats wieder bei einem angestrebten Wert von 60 bis 65 kg/m³h.

## Patentansprüche

1. Verfahren zur kontinuierlichen Hydroformylierung, bei dem ein Olefinausgangsmaterial, welches wenigstens ein Olefin mit 3 bis 20 Kohlenstoffatomen enthält, bei erhöhter Temperatur und bei erhöhtem Druck in einer Reaktionszone mit Synthesegas in Gegenwart eines mit einem Organophosphorliganden komplexierten, homogenen Übergangsmetall-Katalysators und freiem Liganden umgesetzt wird, wobei der Katalysator in situ in der Reaktionszone gebildet wird und man der Reaktionszone zum Ausgleich von Katalysatorverlusten eine Lösung einer Übergangsmetallquelle zufügt, **dadurch gekennzeichnet, dass** man die Raum-Zeit-Ausbeute der Reaktionszone an Hydroformylierungsprodukt bestimmt und die Zugabegeschwindigkeit der Übergangsmetallquelle in die Reaktionszone in Abhängigkeit von der Raum-Zeit-Ausbeute steuert,
wobei man
- ein Stellmittel zum Einstellen der Zugabegeschwindigkeit der Übergangsmetallquelle in die Reaktionszone vorsieht,
- einen Zielwert für die Raum-Zeit-Ausbeute der Reaktionszone an Hydroformylierungsprodukt festlegt,
- den Istwert für die Raum-Zeit-Ausbeute ermittelt,
- nach Erreichen eines unteren Grenzwerts für die Abweichung des Istwerts vom Zielwert die Menge an Übergangsmetallquelle ermittelt, die erforderlich ist, den Katalysatorverlust auszugleichen, und
- der Reaktionszone eine Lösung der Übergangsmetallquelle zufügt, wobei die Zugabegeschwindigkeit der Übergangsmetallquelle in die Reaktionszone so gesteuert wird, dass die Raum-Zeit-Ausbeute einen oberen Grenzwert für die Abweichung des Istwerts vom Zielwert nicht überschreitet.

2. Verfahren nach Anspruch 1, **dadurch gekennzeichnet, dass** der untere Grenzwert für die Abweichung des Istwerts für die Raum-Zeit-Ausbeute vom Zielwert maximal 40 %, besonders bevorzugt maximal 30 %, bezogen auf den Zielwert, beträgt.

3. Verfahren nach Anspruch 1 oder 2, **dadurch gekennzeichnet, dass** der obere Grenzwert für die Abweichung des Istwerts für die Raum-Zeit-Ausbeute vom Zielwert maximal 10 %, besonders bevorzugt maximal 5 %, bezogen auf den Zielwert, beträgt.

4. Verfahren nach einem der vorhergehenden Ansprüche, wobei die Übergangsmetallkonzentration in der Reaktionszone in einem Bereich von 150 bis 250 ppm, besonders bevorzugt von 180 bis 200 ppm, bezogen auf den gesamten flüssigen Inhalt der Reaktionszone, liegt.

5. Verfahren nach einem der vorhergehenden Ansprüche, wobei die Zugabegeschwindigkeit der Übergangsmetallquelle in die Reaktionszone in einem Bereich von 0,1 ppm bis 10 ppm Übergangsmetallquelle pro Tag, bevorzugt in einem Bereich von 0,5 ppm bis 5 ppm Übergangsmetallquelle pro Tag, bezogen auf den gesamten flüssigen Inhalt der Reaktionszone, liegt.

6. Verfahren nach einem der vorhergehenden Ansprüche, **dadurch gekennzeichnet, dass** der durch die Zuführung der Übergangsmetallquelle bedingte Temperaturanstieg in der Reaktionszone weniger als 2 K, bevorzugt weniger als 1 K, insbesondere weniger als 0,5 K, beträgt.

7. Verfahren nach einem der vorhergehenden Ansprüche, **dadurch gekennzeichnet, dass** als Übergangsmetall Rhodium eingesetzt wird.

8. Verfahren nach einem der vorhergehenden Ansprüche, **dadurch gekennzeichnet, dass** die Konzentration der Übergangsmetallquelle in der der Reaktionszone zugeführten Lösung 0,1 bis 10 Gew.-%, besonders bevorzugt 0,2 bis 7,5 Gew.-%, insbesondere 0,5 bis 5 Gew.-%, beträgt.

9. Verfahren nach einem der vorhergehenden Ansprüche, **dadurch gekennzeichnet, dass** ein Rhodiumkatalysator eingesetzt wird, der Triphenylphosphin als Liganden umfasst.

10. Verfahren nach einem der vorhergehenden Ansprüche, **dadurch gekennzeichnet, dass** das Molmengenverhältnis von Ligand zu Übergangsmetall in der Reaktionszone in einem Bereich von 100 : 1 bis 1000 : 1, vorzugsweise 200 : 1 bis 500 : 1, liegt.

11. Verfahren nach einem der vorhergehenden Ansprüche, **dadurch gekennzeichnet, dass** zur Hydroformylierung ein propenhaltiges Kohlenwasserstoffgemisch, insbesondere ein C₃-Schnitt, eingesetzt wird.

12. Verfahren nach einem der Ansprüche 1 bis 11, **dadurch gekennzeichnet, dass** zur Abtrennung des Hydroformylierungsproduktes ein flüssiger Austrag aus der Reaktionszone entnommen wird.

13. Verfahren nach Anspruch 12, **dadurch gekennzeichnet, dass** der Zielwert für die Raum-Zeit-Ausbeute der Reaktionszone an Hydroformylierungsprodukt 75 bis 120 kg/m³h, bevorzugt 85 bis 110 kg/m³h, beträgt.

14. Verfahren nach einem der Ansprüche 1 bis 11, **dadurch gekennzeichnet, dass** zur Abtrennung des Hydroformylierungsproduktes ein gasförmiger Austrag aus der Reaktionszone entnommen wird.

15. Verfahren nach Anspruch 14, **dadurch gekennzeichnet, dass** der Zielwert für die Raum-Zeit-Ausbeute der Reaktionszone an Hydroformylierungsprodukt 50 bis 75 kg/m³h, bevorzugt 55 bis 70 kg/m³h, beträgt.

## Claims

1. A process for continuous hydroformylation, in which an olefin starting material comprising at least one olefin having from 3 to 20 carbon atoms is reacted at elevated temperature and superatmospheric pressure with synthesis gas in the presence of a homogeneous transition metal catalyst complexed with an organophosphorus ligand and free ligand in a reaction zone, where the catalyst is formed in situ in the reaction zone and a solution of a transition metal source is introduced into the reaction zone to compensate catalyst losses, wherein the space-time yield of hydroformylation product in the reaction zone is determined and the rate of introduction of the transition metal source into the reaction zone is controlled as a function of the space-time yield,
where
- an adjusting means for setting the rate of introduction of the transition metal source into the reaction zone is provided,
- a target value for the space-time yield of hydroformylation product in the reaction zone is set down,
- the actual value of the space-time yield is determined,
- after a lower limit for the deviation of the actual value from the target value is reached, the amount of transition metal source required for making up the loss of catalyst is determined and
- a solution of the transition metal source is introduced into the reaction zone, with the rate of introduction of the transition metal source into the reaction zone being controlled so that the space-time yield does not exceed an upper limit for the deviation of the actual value from the target value.

2. The process according to claim 1, wherein the lower limit to the deviation of the actual value of the space-time yield from the target value is not more than 40%, particularly preferably not more than 30%, based on the target value.

3. The process according to claim 1 or 2, wherein the upper limit to the deviation of the actual value of the space-time yield from the target value is not more than 10%, particularly preferably not more than 5%, based on the target value.

4. The process according to any of the preceding claims, wherein the transition metal concentration in the reaction zone is in the range from 150 to 250 ppm, particularly preferably from 180 to 200 ppm, based on the total liquid content of the reaction zone.

5. The process according to any of the preceding claims, wherein the rate of introduction of the transition metal source into the reaction zone is in the range from 0.1 ppm to 10 ppm of transition metal source per day, preferably in the range from 0.5 ppm to 5 ppm of transition metal source per day, based on the total liquid content of the reaction zone.

6. The process according to any of the preceding claims, wherein the temperature increase in the reaction zone brought about by the introduction of the transition metal source is less than 2 K, preferably less than 1 K, in particular less than 0.5 K.

7. The process according to any of the preceding claims, wherein the rhodium is used as transition metal.

8. The process according to any of the preceding claims, wherein the concentration of the transition metal source in the solution introduced into the reaction zone is from 0.1 to 10% by weight, particularly preferably from 0.2 to 7.5% by weight, in particular from 0.5 to 5% by weight.

9. The process according to any of the preceding claims, wherein a rhodium catalyst comprising triphenylphosphine as ligand is used.

10. The process according to any of the preceding claims, wherein the molar ratio of ligand to transition metal in the reaction zone is in the range from 100:1 to 1000:1, preferably from 200:1 to 500:1.

11. The process according to any of the preceding claims, wherein a propene-comprising hydrocarbon mixture, in particular a C₃ fraction, is used for the hydroformylation.

12. The process according to any of claims 1 to 11, wherein a liquid discharge is taken off from the reaction zone to separate off the hydroformylation product.

13. The process according to claim 12, wherein the target value for the space-time yield of hydroformylation product in the reaction zone is from 75 to 120 kg/m³h, preferably from 85 to 110 kg/m³h.

14. The process according to any of claims 1 to 11, wherein a gaseous discharge is taken off from the reaction zone to separate off the hydroformylation product.

15. The process according to claim 14, wherein the target value for the space-time yield of hydroformylation product in the reaction zone is from 50 to 75 kg/m³h, preferably from 55 to 70 kg/m³h.

## Revendications

1. Procédé d'hydroformylation continue, selon lequel un matériau de départ oléfinique, qui contient au moins une oléfine de 3 à 20 atomes de carbone, est mis en réaction à température élevée et à pression élevée dans une zone de réaction avec un gaz de synthèse en présence d'un catalyseur de métal de transition homogène complexé avec un ligand organophosphore et d'un ligand libre, le catalyseur étant formé in situ dans la zone de réaction et une solution d'une source de métal de transition étant introduite dans la zone de réaction pour compenser les pertes de catalyseur, **caractérisé en ce que** le rendement espace-temps de la zone de réaction en produit d'hydroformylation est déterminé et la vitesse d'ajout de la source de métal de transition dans la zone de réaction est réglée en fonction du rendement espace-temps,
- un moyen de réglage étant prévu pour l'ajustement de la vitesse d'ajout de la source de métal de transition dans la zone de réaction,
- une valeur cible pour le rendement espace-temps de la zone de réaction en produit d'hydroformylation étant fixée,
- la valeur réelle pour le rendement espace-temps étant déterminée,
- après avoir atteint une valeur limite inférieure pour la déviation de la valeur réelle de la valeur cible, la quantité de source de métal de transition nécessaire pour compenser la perte de catalyseur étant déterminée, et
- une solution de la source de métal de transition étant ajoutée à la zone de réaction, la vitesse d'ajout de la source de métal de transition dans la zone de réaction étant réglée de sorte que le rendement espace-temps ne dépasse pas une valeur limite supérieure pour la déviation de la valeur réelle de la valeur cible.

2. Procédé selon la revendication 1, **caractérisé en ce que** la valeur limite inférieure pour la déviation de la valeur réelle pour le rendement espace-temps de la valeur cible est d'au plus 40 %, de manière particulièrement préférée d'au plus 30 %, par rapport à la valeur cible.

3. Procédé selon la revendication 1 ou 2, **caractérisé en ce que** la valeur limite supérieure pour la déviation de la valeur réelle pour le rendement espace-temps de la valeur cible est d'au plus 10 %, de manière particulièrement préférée d'au plus 5 %, par rapport à la valeur cible.

4. Procédé selon l'une quelconque des revendications précédentes, **caractérisé en ce que** la concentration en métal de transition dans la zone de réaction se situe dans une plage allant de 150 à 250 ppm, de manière particulièrement préférée de 180 à 200 ppm, par rapport au contenu liquide total de la zone de réaction.

5. Procédé selon l'une quelconque des revendications précédentes, **caractérisé en ce que** la vitesse d'ajout de la source de métal de transition dans la zone de réaction se situe dans une plage allant de 0,1 ppm à 10 ppm de source de métal de transition par jour, de préférence dans une plage allant de 0,5 ppm à 5 ppm de source de métal de transition par jour, par rapport au contenu liquide total de la zone de réaction.

6. Procédé selon l'une quelconque des revendications précédentes, **caractérisé en ce que** l'élévation de température provoquée par l'introduction de la source de métal de transition dans la zone de réaction est de moins de 2 K, de préférence de moins de 1 K, notamment de moins de 0,5 K.

7. Procédé selon l'une quelconque des revendications précédentes, **caractérisé en ce que** du rhodium est utilisé en tant que métal de transition.

8. Procédé selon l'une quelconque des revendications précédentes, **caractérisé en ce que** la concentration de la source de métal de transition dans la solution introduite dans la zone de réaction est de 0,1 à 10 % en poids, de manière particulièrement préférée de 0,2 à 7,5 % en poids, notamment de 0,5 à 5 % en poids.

9. Procédé selon l'une quelconque des revendications précédentes, **caractérisé en ce qu'**un catalyseur de rhodium est utilisé, qui comprend de la triphénylphosphine en tant que ligand.

10. Procédé selon l'une quelconque des revendications précédentes, **caractérisé en ce que** le rapport entre les quantités molaires du ligand et du métal de transition dans la zone de réaction se situe dans une plage allant de 100:1 à 1 000:1, de préférence de 200:1 à 500:1.

11. Procédé selon l'une quelconque des revendications précédentes, **caractérisé en ce qu'**un mélange d'hydrocarbures contenant du propène, notamment une coupe en C₃, est utilisé pour l'hydroformylation.

12. Procédé selon l'une quelconque des revendications 1 à 11, **caractérisé en ce qu'**une sortie liquide est soutirée de la zone de réaction pour la séparation du produit d'hydroformylation.

13. Procédé selon la revendication 12, **caractérisé en ce que** la valeur cible pour le rendement espace-temps de la zone de réaction en produit d'hydroformylation est de 75 à 120 kg/m³h, de préférence de 85 à 110 kg/m³h.

14. Procédé selon l'une quelconque des revendications 1 à 11, **caractérisé en ce qu'**une sortie gazeuse est soutirée de la zone de réaction pour la séparation du produit d'hydroformylation.

15. Procédé selon la revendication 14, **caractérisé en ce que** la valeur cible pour le rendement espace-temps de la zone de réaction en produit d'hydroformylation est de 50 à 75 kg/m³h, de préférence de 55 à 70 kg/m³h.
